# FASCICULE DE BREVET EUROPEEN

(11) **EP 3 849 671 B1**
(45) Date de publication et mention de la délivrance du brevet: **24.04.2024**
(21) Numéro de dépôt: 19774168.9
(22) Date de dépôt: 03.09.2019
(51) Int. Cl.: A61Q 17/00, A61K 8/96

(54) **UTILISATION D'EAU DE REOTIER POUR LIMITER LA PENETRATION CUTANEE DES POLLUANTS**
VERWENDUNG VON REOTIER WASSER ZUR BEGRENZUNG DER HAUTPENETRATION VON SCHADSTOFFEN
USE OF REOTIER WATER TO LIMIT SKIN PENETRATION OF POLLUTANTS

(30) Priorité: 14.09.2018 FR 1858329
(43) Date de publication de la demande: 21.07.2021
(73) Titulaire: LABORATOIRES M & L, 04100 Manosque (FR)
(72) Inventeur: CENIZO, Valérie, 13650 MEYRARGUES (FR); PORTES, Pascal, 13540 PUYRICARD (FR)
(74) Mandataire: Renard, Emmanuelle
(86) Numéro de dépôt international: PCT/FR2019/052022
(87) Numéro de publication internationale: WO 2020/053503

(56) Documents cités:
- DATABASE GNPD [Online] MINTEL; 20 mars 2018 (2018-03-20), anonymous: "Ultra-Thirst Quenching Cream", XP055592713, extrait de www.gnpd.com Database accession no. 5461797
- DATABASE GNPD [Online] MINTEL; 31 juillet 2018 (2018-07-31), anonymous: "Fresh Moisturizing Mist", XP055592695, extrait de www.gnpd.com Database accession no. 5867249
- DATABASE GNPD [Online] MINTEL; 12 juillet 2018 (2018-07-12), anonymous: "Water Gel Cleanser", XP055592697, extrait de www.gnpd.com Database accession no. 5825515
- DATABASE GNPD [Online] MINTEL; 20 mars 2018 (2018-03-20), anonymous: "Moisture Prep Essence", XP055592716, extrait de www.gnpd.com Database accession no. 5461795

## Description

La présente invention concerne une méthode cosmétique pour freiner ou limiter la pénétration d'agents polluants dans la peau.

### Arrière-plan technologique

Dans son environnement quotidien, et spécialement dans un environnement urbain, la peau d'un individu est soumise à de nombreuses agressions de polluants atmosphériques.

Les principaux polluants urbains qui peuvent avoir un effet délétère sur la peau sont les gaz toxiques, les métaux lourds, les hydrocarbures polycycliques aromatiques (HPA) et des particules qui sont des résidus de combustion sur lesquels une grande quantité de composés organiques et minéraux sont adsorbés.

Les gaz toxiques tels que l'ozone, le monoxyde de carbone, les oxydes d'azote ou de soufre favorisent ainsi la desquamation des matériaux composés de kératine, tels que la peau ou les cheveux ; ils les rendent ternes et sales. Des métaux tels que le mercure, le cadmium ou le plomb sont aussi connus pour être des polluants atmosphériques dont les émissions ont considérablement augmenté. Or, certains métaux peuvent pénétrer dans la peau et s'y accumuler.

Les hydrocarbures polycycliques aromatiques, typiquement adsorbés à la surface de particules issues de combustion ainsi que sur la poussière provenant de l'atmosphère urbaine, peuvent pénétrer dans la peau, s'y accumuler ou s'y biotransformer.

Ces polluants peuvent être à l'origine d'interférences dans le fonctionnement normal des lipides, de l'ADN ou bien des protéines de la peau humaine, et favorisent le vieillissement de la peau par un effet d'oxydation. Ils affectent également le teint ; le monoxyde de carbone va se mélanger à l'oxygène dans le sang et ainsi provoquer un changement de couleur de la peau, qui paraîtra plus grisâtre. Enfin, une forte exposition aux gaz d'échappements ou aux fumées polluantes ainsi qu'à des polluants d'intérieur tels que la fumée de cigarette ou encore le chauffage, peuvent mener à un dessèchement cutané et à des irritations.

### Résumé de l'invention

Les inventeurs ont maintenant identifié de nouvelles propriétés de l'eau de Réotier, qu'ils proposent de mettre à profit dans une application cosmétique anti-pollution.

Un objet de l'invention est ainsi une méthode cosmétique pour limiter la pénétration cutanée d'un ou plusieurs agent(s) polluant(s), comprenant l'application sur la peau d'une composition cosmétique comprenant, ou constituée par, de l'eau de Réotier.

L'effet est particulièrement rapide et permet une protection immédiate contre la pénétration des agents polluants.

L'invention vise donc l'utilisation cosmétique d'eau de Réotier comme agent anti-pollution.

Selon l'invention, l'application topique d'eau de Réotier permet de lutter contre les effets néfastes de la pollution sur la peau, y compris les dommages cutanés dus à la présence d'oxydants et de radicaux libres, et un teint terne ou un manque d'éclat.

L'eau de Réotier peut ainsi être utilisée, par application topique sur la peau, pour prévenir ou traiter l'altération de la structure épidermique de la peau causée par la pollution.

L'eau de Réotier peut être utilisée directement sous forme de brume pulvérisée, ou sous toute forme adaptée à une application topique sur la peau et notamment sous forme de lait, de crème, de lotion, de gel, de masque, de pâte ou de film. L'eau de Réotier peut être notamment incluse dans une composition non rincée et en particulier une composition de soin, une composition de maquillage, telle qu'un fond de teint, ou une composition solaire.

### Légende des figures

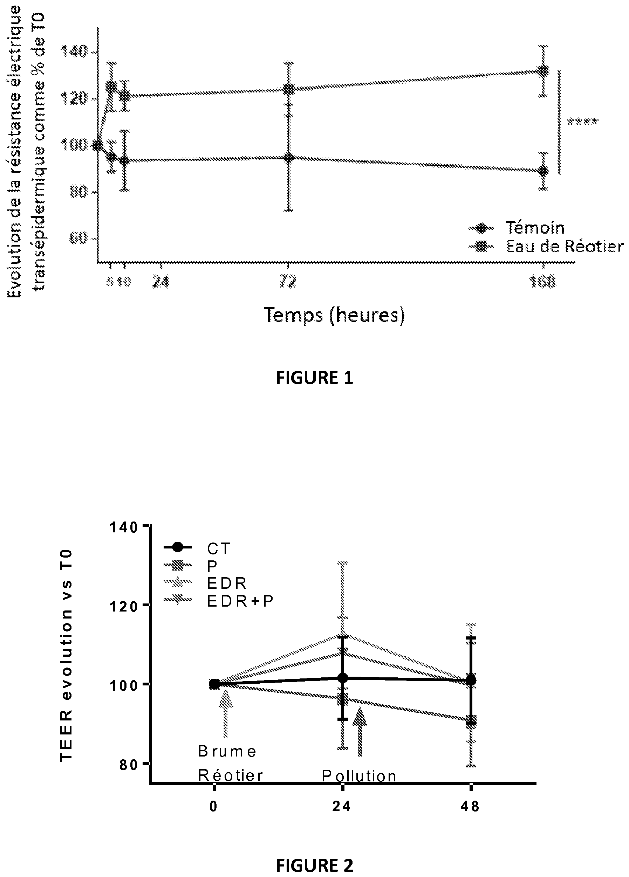

**Figure 1****. Evolution cinétique (heures) de l'impédance d'explants après traitement** à **l'eau de Réotier *vs* un explant non traité**. Les données ont été normalisées par rapport à l'explant de peau témoin à T0. Les données représentent la moyenne ± les valeurs de déviation standard de 3 donneurs en quadruple et ont été analysées par analyse de variance (Anova) à deux facteurs (****p< 0.0001).
**Figure 2****. Evolution cinétique moyenne (heures) de l'impédance d'explants de peau des trois donneurs après traitement à l'eau de Réotier et application de polluants.** Une mesure d'impédance est prise sur les explants (T0) puis la brume Réotier est appliquée sur certains explants (EDR). Après 24h (T24) une nouvelle mesure d'impédance est effectuée puis un mélange de polluants est appliqué en surface des explants. Une dernière mesure d'impédance est prise sur les explants à 48h (T48). Quatre explants de chaque donneur ont été utilisés avec un explant contrôle (CT), un explant sur lequel ont été appliqués uniquement les polluants (P), un explant sur lequel seulement l'eau de Réotier a été appliquée (EDR) et enfin un explant sur lequel l'eau de Réotier puis les polluants ont été appliqués (EDR + P).

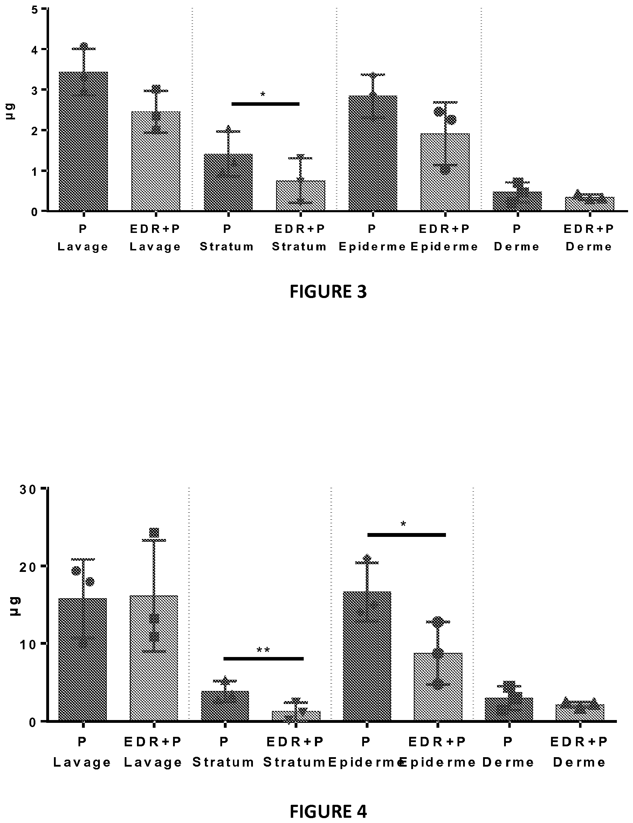

**Figure 3****. Dosage HPLC du polluant Dibenzoanthracène sur des échantillons de peau** : **la surface, le stratum corneum, l'épiderme et le derme.** Mesure témoin avec seulement le polluant (P) et mesure sur les explants de peau où l'eau de Réotier a été appliquée (EDR + P).
**Figure 4****. Dosage HPLC du polluant Benzo[a]pyrène sur des échantillons de peau : la surface, le stratum corneum, l'épiderme et le derme.** Mesure témoin avec seulement le polluant (P) et mesure sur les explants de peau où L'eau de Réotier a été appliquée (EDR + P).

### Description détaillée de l'invention

### L'eau de Réotier

L'eau de Réotier est une eau naturelle d'origine profonde avec une composition physico-chimique constante. La fontaine de Réotier se situe en France dans les Hautes-Alpes, dans la commune de Réotier, à 910 m d'altitude environ. Les eaux qui s'écoulent des sources chaudes à proximité s'infiltrent dans le sol avant de remonter, enrichies de minéraux. La composition constante de l'eau de Réotier est décrite dans le tableau ci-dessous.

**Tableau : Composition ionique de l'eau de Réotier (mg/L)**

| **Composition** | **mg/L** |
|---|---|
| Résidus secs à 180°C | 4121 |
| Sulfates | 1503 |
| Chlorure | 943 |
| Nitrates | 0,1 |
| Orthophosphates | 0,08 |
| Calcium | 599 |
| Magnésium | 73 |
| Sodium | 683 |
| Potassium | 18 |
| Fer | 0,011 |
| Manganèse | 0,014 |
| Strontium | 11,2 |
| Aluminium | < 0,005 |
| Silicium | 11,2 |
| Lithium | 0,8 |
| Sélénium | < 0,005 |
| Cuivre | < 0,005 |
| Zinc | < 0,005 |
| Bore | 1,5 |

L'eau de Réotier est ainsi caractérisée par un contenu minéral élevé notamment en calcium, sodium, magnésium et strontium.

### Formulations galéniques

L'eau de Réotier peut être appliquée sous la forme de brume (pulvérisable typiquement au moyen de brumisateurs ou vaporisateurs), mais aussi sous n'importe quelle forme galénique appropriée à une application topique ou cutanée, éventuellement en mélange avec des excipients cosmétiquement acceptables.

Les compositions cosmétiques ainsi formulées peuvent typiquement comprendre de 1 à 100% en poids d'eau de Réotier. Par exemple il peut s'agir d'une brume à 100% d'eau de Réotier, ou d'une composition comprenant de 1 à 95%, de préférence de 1 à 90%, de préférence de 1 à 80%, de préférence encore 1 à 70%, 1 à 60%, 1 à 50%, 1 à 40%, 1 à 30%, 1 à 20%, 1 à 10%, ou 1 à 5% en poids, d'eau de Réotier. Dans un mode de réalisation préféré, la composition galénique comprend de 1 à 30% ou 1 à 10% en poids d'eau de Réotier.

On peut ainsi utiliser une formulation solide, liquide ou semi-solide comme des crèmes, des laits, des baumes, des mousses, des lotions, des sérums, des gels ou des gels crème. L'eau de Réotier peut ainsi être formulée sous forme d'émulsions huile-dans-eau ou eau-dans-huile, d'émulsions multiples, de solutions aqueuses, hydro-alcooliques ou hydro-glycoliques ou de dispersions aqueuses. L'eau de Réotier peut être incorporée dans différents produits destinés au soin et/ou au maquillage de la peau, tels que des soins hydratants ou des fonds de teint, ou au nettoyage de la peau, tels que des lotions, gels ou laits démaquillants, des masques, ou encore dans des produits de soin et/ou de lavage des cheveux, tels que des shampooings et après-shampooings, entre autres.

Dans un mode de réalisation particulier, la composition cosmétique est sous la forme d'un gel aqueux ou d'une émulsion huile-dans-eau.

La phase aqueuse renferme alors l'eau de Réotier et éventuellement de l'eau déionisée et/ou au moins un constituant choisi parmi les polyols et les gélifiants aqueux. L'eau représente au total avantageusement de 40 à 95%, de préférence 40 à 90% du poids total de la composition. Le polyol peut notamment être choisi parmi la glycérine, le propylène glycol, le butylène glycol, le pentylène glycol et leurs mélanges et il peut représenter de 5 à 30% du poids total de la composition.

Dans un mode de réalisation particulier, la composition cosmétique est un gel comprenant de 40 à 95% en poids d'eau de Réotier.

Par "gélifiant aqueux", on désigne un composé polymérique capable d'immobiliser des molécules d'eau en s'hydratant et d'augmenter ainsi la viscosité de la phase aqueuse. Un tel gélifiant peut être choisi parmi : les polysaccharides, tels que : la cellulose et ses dérivés, les amidons modifiés, le carraghénane, l'agar agar, la gomme de xanthane et les gommes végétales telles que la gomme de guar ou de caroube ; les polymères synthétiques et notamment les copolymères d'acide 2-acrylamido-2-méthylpropane sulfonique (AMPS) avec la vinylpyrrolidone, les homopolymères d'acrylate de sodium éventuellement réticulés, ainsi que les copolymères acryliques, en particulier les copolymères d'acrylate de sodium et ses dérivés, et/ou de (méth)acrylate d'alkyle et/ou de (méth)acrylate d'hydroxyalkyle et/ou de (méth)acrylate de (polyéthoxy)alkyle, avec éventuellement au moins un autre monomère, avantageusement l'acide 2-acrylamido-2-méthylpropane sulfonique (AMPS), ces copolymères étant éventuellement réticulés ; et leurs mélanges.

Lorsqu'elle est présente, la phase grasse peut comprendre une ou plusieurs huiles volatiles et/ou non volatiles. Des exemples d'huiles volatiles sont les alcanes ramifiés, tels que l'isododécane, et les alcanes linéaires en C10-C13. Comme huiles non volatiles, on peut citer notamment :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane (C30), notamment le squalane végétal extrait de l'huile d'olive, et l'hémisqualane (C15) ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le caprylate de propylheptyle et leurs mélanges. D'autres esters utilisables sont les diesters d'acides et de monoalcools tels que l'adipate de disopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

La phase grasse peut en outre comprendre au moins un structurant de phase grasse. Par "structurant de phase grasse", on entend un composé capable d'épaissir les huiles contenues dans la composition, choisi notamment parmi les cires, les gélifiants de phase grasse et les corps gras pâteux, ainsi que leurs mélanges.

La composition peut renfermer en outre au moins un actif cosmétique, en particulier un autre agent anti-polluant, tel que des polymères filmogènes à base de polysaccharides, capables de former un film protecteur anti-pollution, en particulier les produits commercialisés par SOLABIA sous les dénominations commerciales Pollustop^{®} et Solashield^{®}, un agent humectant, tel que l'acide hyaluronique, l'urée ou le mélange de xylityl glucoside, xylitol et anhydroxylitol commercialisé par SEPPIC sous la dénomination commerciale Aquaxyl^{®} ; un agent desquamant, tel que des α- et β-hydroxyacides ; et leurs mélanges.

La composition peut en outre contenir différents constituants qui peuvent être dispersés dans la phase grasse et/ou dans la phase aqueuse de l'émulsion, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% et de préférence de 4 à 6% du poids total de la composition.

La composition peut en outre comprendre des additifs choisis notamment parmi des agents photoprotecteurs organiques et/ou inorganiques, actifs dans la lumière bleue et/ou l'UVA et/ou l'UVB ; des particules exfoliantes ; des parfums ; des agents anti-oxydants ; des agents séquestrants ; des ajusteurs de pH ; des conservateurs ; des charges ; des pigments ; des colorants ; et leurs mélanges.

La composition utilisée selon l'invention peut être appliquée sur au moins une zone du corps d'une personne exposée à des agents polluants, et plus particulièrement sur le visage, les lèvres, le cou et/ou le décolleté. En variante ou en plus, elle peut être appliquée sur les mains ou les bras, sur les demi jambes et les pieds.

Cette composition peut être appliquée une ou plusieurs fois par jour, par exemple matin et/ou soir, sur les zones visées.

### Agents polluants

Les agents polluants incluent principalement les polluants atmosphériques.

Les polluants atmosphériques désignent les polluants présents dans l'environnement, qui se présentent notamment sous forme de gaz et de particules respirables. Ils peuvent être présents à l'extérieur, par exemple les particules de moteur diesel, l'ozone, ou les métaux lourds, et/ou à l'intérieur des maisons où la pollution peut notamment être due à la fumée de cigarette ou aux solvants libérés par les peintures, les colles ou les papiers peints, comme le toluène, styrène, xylène, benzaldéhyde.

Parmi les polluants présents dans l'environnement, on distingue les polluants primaires qui sont directement issus des sources de pollution (trafic routier, industries, chauffage, agriculture...) et les polluants secondaires qui proviennent de réactions chimiques de gaz entre eux. Parmi les polluants primaires, on peut citer les oxydes de carbone (CO et CO₂), les oxydes d'azote (par exemple NO, NO₂), les oxydes de soufre (par exemple SO, SO₂), les hydrocarbures, plus particulièrement les hydrocarbures polycycliques aromatiques (HPA), les composés organiques volatils, les métaux (tels que plomb, mercure, cadmium) et les particules fines ou ultrafines, telles que les PM10 (de diamètre inférieur à 10 µm) et les PM2,5 (de diamètre inférieur à 2,5 µm). Ces dernières peuvent être particulièrement toxiques par leur capacité à pénétrer profondément, leur taille étant inférieure à la taille des pores de la peau humaine. Les microparticules PM2,5 sont produites par les moteurs diesel, la combustion du charbon et la fumée de cigarette.

L'invention permet de limiter (freiner ou réduire) la pénétration cutanée d'un ou plusieurs agent(s) polluant(s), par application d'eau de Réotier sur la peau.

L'invention est particulièrement utile pour limiter la pénétration des HPA, tels que le benzopyrène, et des microparticules (notamment les particules PM10 et PM2,5).

Les exemples ci-dessous sont donnés à titre purement illustratif et n'ont pas pour but de limiter la portée de l'invention.

### Exemples

### Exemple 1 : Formulations

Des exemples de formulations sont présentés ci-dessous (pourcentages exprimés en poids de la composition).

### Exemple de crème :

| | |
|---|---|
| EAU DE REOTIER | 1 - 10% |
| AUTRES ACTIFS | 5 - 13% |
| EMOLLIENT | 5 - 15% |
| EMULSIONNANT | 1 - 5% |
| GELIFIANT AQUEUX | 1 - 5% |
| POLYOL | 3 - 8% |
| Eau déionisée | 60 - 70% |
| AJUSTEUR DE PH | QS |
| ANTIOXYDANT | QS |
| CONSERVATEUR | QS |
| PARFUM | QS |
| PIGMENT | QS |
| SEQUESTRANT | QS |

### Exemple de gel hydratant :

| | |
|---|---|
| EAU DE REOTIER | 1 - 10% |
| AUTRES ACTIFS | 5 - 13% |
| GELIFIANT AQUEUX | 1 - 3% |
| POLYOL | 10-15% |
| SOLUBILISANT | 0.1 - 1% |
| Eau déionisée | 70 - 80% |
| AJUSTEUR DE PH | QS |
| PARFUM | QS |
| PIGMENT | QS |

### Exemple 2 : Evaluation de la résistance électrique d'explants cutanés (impédancemétrie)

### Matériel et méthodes

### Mesure de l'impédance :

- Principe : Deux électrodes sont placées de part et d'autre d'un disque de peau avant l'application d'un courant électrique (100 Hz). L'impédance mesure la résistance de la peau au passage du courant électrique. Plus la peau est lésée, plus sa résistance au courant électrique est faible. Elle est mesurée en Ω.
- Mode opératoire : Chaque fragment est déposé sur la première électrode surmontée d'un coton imbibé de solution saline (PBS). 100 µl de PBS sont déposés à la surface de la peau. La deuxième électrode est alors mise en contact du PBS, fermant ainsi le circuit électrique et permettant la mesure.

Des explants cutanés de 3 donneurs différents (femmes de 42, 55 et 55 ans) ont été traités le jour de l'opération. Pour chacune des conditions testées, 4 disques de 2,2 cm de diamètre ont été ponctionnés sur la peau de chaque donneuse (n=12 par condition). L'eau de Réotier a été pulvérisée au-dessus de la moitié des explants de peau. Les explants de peau ont ensuite été placés sur une plaque de culture à 6 puits, à l'interface air-liquide. Le milieu de culture a été renouvelé tous les deux jours et une nouvelle pulvérisation d'eau de Réotier a été effectuée. Les mesures de résistance électrique transépidermique sont effectuées après 5h, 10h, 3 ou 7 jours.

### Résultats

Les résultats de mesure de la résistance électrique transépidermique des explants de peau sont présentés à la **Figure 1****.**

Etonnamment, après seulement un traitement avec de l'eau de Réotier, la résistance électrique transépidermique augmente rapidement de 30% 5 heures après le traitement et ensuite se stabilise jusqu'au jour 7 (168 heures, P < 0,0001). Au contraire, les explants cutanés témoins ont montré une légère diminution de la résistance électrique transépidermique dans la culture qui s'est ensuite stabilisée après 10 heures (89% de la valeur T0 au jour 7).

### Exemple 3 : Evaluation de l'effet de l'eau de Réotier sur l'impédance en présence d'agents polluants

### Matériel et méthodes

Afin de tester l'efficacité de l'eau de Réotier vis-à-vis de la pénétration des polluants, un test d'application de polluants sur des explants de peau a été réalisé. Certains explants ont été vaporisés avec une brume d'eau de Réotier, 24h avant l'application de polluants. A T0, 24 et 48h, la résistance électrique de la peau (impédancemétrie) a été mesurée, reflétant la pénétration des polluants dans les différentes couches de la peau.

### 1. Fragments de peau

Trois explants de peau sont utilisés pour chaque partie dans l'heure suivant l'opération : Les explants sont débarrassés de l'hypoderme à l'aide d'un scalpel. La surface de la peau est délicatement nettoyée avec de l'eau distillée afin d'éliminer les traces de sang.

### 2. Préparation du polluant

Un mélange de polluants est préparé à partir de différentes solutions :
- Solution de PM 2,5 (particules fines de diamètre inférieur à 2,5 µm)
- Solution PM10 (particules fines de diamètre inférieur à 10 µm)
- Solution de benzoαpyrène (2 mg/ml)
- Solution de dibenzanthracène (1 mg/ml)
- Benzène

Les solutions de PM 2,5 et 10 sont obtenues par extraction des disques de prélèvement dans de l'eau distillée (24 disques/40 ml).

### Mélange des solutions :

- 12,5 µl de solution de PM 2,5
- 12,5 µl de solution de PM10
- 25 µl de solution de benzoαpyrène (2 mg/ml)
- 25 µl de solution de dibenzanthracène (1 mg/ml)
- 10 µl de benzène

### 3. Etude de la viabilité, de l'impédance et histologie

### 3.1. Préparation des explants

12 disques de 2.2 cm de diamètre sont obtenus à l'aide d'un emporte-pièce. Les explants sont placés dans un bain contenant des antibiotiques et des antifongiques pendant 30 minutes. Ils sont placés dans des inserts de culture de plaque 6 puits contenant 2,5 ml de milieu de culture spécifique et déposés dans un incubateur à 37°C.

### 3.2. Traitement des explants (T0)

Le lendemain, chaque explant est déposé sur une lingette. L'impédance est mesurée. La brume d'eau de Réotier est appliquée après les mesures par 3 pulvérisations successives. Les disques sont replacés dans les inserts de culture contenant du milieu neuf et laissés sous la hotte couvercle ouvert pendant 15 minutes. Lorsque les explants sont secs, ils sont placés dans l'incubateur à 37°C avec 5% de CO2.

Quatre groupes sont ainsi constitués :
- Témoin : pas de brume, pas de pollution
- Pollution : pas de brume, dépôt de pollution
- Brume Réotier : pulvérisation de la brume d'eau de Réotier, pas de pollution
- Pollution + brume Réotier : pulvérisation de la brume d'eau de Réotier, dépôt de pollution.

### 3.3. Traitement des explants (T24h)

Le lendemain, chaque explant est déposé sur une lingette. L'impédance est mesurée. La brume Réotier est appliquée après les mesures par 3 pulvérisations successives. Après 15 minutes sous la hotte, le mélange de polluants est ajouté sur les disques (85 µl). Les explants sont placés dans les inserts puis dans l'incubateur.

### 3.4. Traitement des explants (T48h)

Le lendemain, chaque explant est déposé sur une lingette. L'impédance est mesurée. Un emporte-pièce (« punch ») de 3 mm de diamètre est déposé dans un tube Eppendorf pour la mesure de la viabilité cellulaire.

### 4. Etude de la pénétration cutanée

### 4.1. Préparation des explants

La peau de 3 donneurs est dermatomée (c'est-à-dire où l'épaisseur de la peau a été réduite à l'épiderme et à une partie du derme) puis 7 disques de 2.2 cm de diamètre sont obtenus à l'aide d'un emporte-pièce. Les disques sont montés dans le système K-Skin^{®} de la société Proviskin après leur mesure d'épaisseur. K-Skin^{®} est un kit de peau d'origine humaine prêt à l'emploi, qui est composé d'un insert en polymère médical de forme circulaire. Les disques sont ensuite placés dans le système D-skin^{®} de la société Proviskin en présence de milieu de culture spécifique.

### 4.2. Traitement des explants (T0)

Le lendemain, chaque K-skin est déposé sur une lingette. La brume d'eau de Réotier est appliquée par 3 pulvérisations successives. Les disques sont replacés dans le système D-skin contenant du milieu neuf et laissés sous la hotte couvercle ouvert pendant 15 minutes. Lorsque les explants sont secs, ils sont placés dans l'incubateur à 37°C avec 5% de CO2.

Quatre groupes sont ainsi constitués :
- Témoin : pas de brume, pas de pollution (n=3)
- Pollution : pas de brume, dépôt de pollution (n=3)
- Brume Réotier : pulvérisation de la brume d'eau de Réotier, pas de pollution (n=3)
- Pollution + brume Réotier : pulvérisation de la brume d'eau de Réotier, dépôt de pollution (n=3)

### 4.3. Traitement des explants (T24h)

Le lendemain, chaque K-skin est déposé sur une lingette. La brume Réotier est appliquée par 3 pulvérisations successives. Après 15 minutes sous la hotte, le mélange de polluants est ajouté sur les disques (85 µl). Le milieu de culture est remplacé par du PBS contenant 6% de PEG. Les K-skin sont placés dans le D-skin puis dans l'incubateur. Après 4h, 6h, 8h, 10h, 12h et 24h de culture, 500 µl de milieu sont prélevés pour les dosages des polluants. Les échantillons sont conservés à -20°C jusqu'aux dosages. 500 µl de PBS sont rajoutés dans les puits.

### 4.4. Traitement des explants (T48h)

Après le dernier prélèvement, un coton tige est appliqué à la surface de chaque peau afin de prélever la quantité de polluant en surface de la peau. Un disque adhésif D-squame^{®} est appliqué sur la surface de chaque disque. Chaque disque adhésif de stratum corneum est placé dans un tube de 20 ml en verre. La séparation dermo-épidermique est effectuée à l'aide d'une pince. Chaque tissu ainsi séparé est pesé puis conservé à -20°C jusqu'à son traitement.

### Résultats

La mesure d'impédancemétrie (ou TEER pour "transepidermal electrical résistance") permet d'évaluer la perméabilité paracellulaire de l'épiderme, c'est-à-dire la circulation des fluides et ions entre les cellules de l'épiderme. L'augmentation de cette résistance est corrélée avec une meilleure fonctionnalité des jonctions serrées et une plus grande imperméabilité de l'épiderme. La moyenne des résultats obtenus sur des explants de peau provenant de 3 donneurs est présentée à la **Figure 2****.** Après 24h d'équilibration à l'incubateur, une mesure d'impédance est prise sur les explants (T0) puis la brume d'eau de Réotier est appliquée sur certains explants (EDR). Après 24h (T24) une nouvelle mesure d'impédance a été effectuée. Elle révèle une légère augmentation de la résistance électrique des explants suite à l'application de la brume. Puis après la mesure à T24, un mélange de polluants est appliqué en surface des explants les explants sont mis en culture, puis une mesure d'impédance à 48h (T48) est réalisée. Une analyse de la variance à 2 facteurs (ANOVA) a été réalisée sur la moyenne des 3 donneurs **(****Fig. 2****).** Les résultats suivants peuvent être observés :
- La pollution fait chuter les valeurs d'impédance. Ce résultat est significatif entre les temps P-T48 et P-T0(*p<0.05). Cette chute d'impédance n'est pas observée sur les peaux contrôles, les peaux traitées par l'eau de Réotier ou les peaux traitées par l'eau de Réotier sur lesquelles ont été appliqués des polluants.
- Le traitement à l'eau de Réotier entraîne une augmentation de l'impédancemétrie : en effet EDR-T24h est significativement différent de EDR-T0 (**p<0.01)

Ces résultats montrent que le traitement préalable des peaux à l'eau de Réotier prévient la perturbation de la barrière cutanée causée par les polluants et visualisée par une chute de l'impédance cutanée.

### Exemple 4 : Evaluation de l'effet de l'eau de Réotier sur la pénétration des polluants dans les différentes couches de la peau par chromatographie liquide haute performance (HPLC)

### Matériel et méthodes

L'impédance est ici mesurée dans les mêmes conditions opératoires que dans l'Exemple 1.

Une extraction des polluants est effectuée pour les échantillons correspondant à la surface, au stratum corneum, à l'épiderme et au derme. 1,5 ml de solvant d'extraction (acétone/eau, 50/50) sont placés dans chaque tube avant d'être mis sous agitation pendant 24h. Les échantillons sont alors filtrés avant d'être injectés dans la colonne d'HPLC.
- Conditions analytiques : Les conditions analytiques sont les suivantes :
   - Phase mobile : eau/acétonitrile (gradient)
   - Colonne : C18, 5µm 25 cm x 4,6 mm
   - Débit : 1,2 ml/min
   - Volume injection : 20 µl
   - T°C : 30°C
   - Longueur d'onde : 254 nm

### Résultats

La **Figure 3** présente les résultats de dosage HPLC du polluant dibenzoanthracène dans les différentes couches de la peau. Un test statistique est réalisé (t-test apparié (*p<0.05)).

**Tableau : Dosage du dibenzoanthracène (en µg)**

| | **Lavage** | | **Stratum** | | **Epiderme** | | **Derme** | |
|---|---|---|---|---|---|---|---|---|
| | **Pollution** | **Brume + pollution** | **Pollution** | **Brume + pollution** | **Pollution** | **Brume + pollution** | **Pollution** | **Brume + pollution** |
| **Donneur 1** | 2,93 | 3,02 | 1,22 | 0,73 | 2,88 | 2,46 | 0,71 | 0,31 |
| **Donneur 2** | 4,07 | 2,35 | 0,99 | 0,21 | 3,35 | 1,03 | 0,47 | 0,42 |
| **Donneur 3** | 3,30 | 2,01 | 2,04 | 1,32 | 2,30 | 2,26 | 0,21 | 0,29 |

La **Figure 4** présente les résultats de dosage HPLC du polluant benzo*α*pyrène dans les différentes couches de la peau. Un test statistique est réalisé (t-test apparié (*p<0.05 et **p<0.01)).

**Tableau : Dosage du benzopyrène (en µg)**

| | **Lavage** | | **Stratum** | | **Epiderme** | | **Derme** | |
|---|---|---|---|---|---|---|---|---|
| | **Pollution** | **Brume + pollution** | **Pollution** | **Brume + pollution** | **Pollution** | **Brume + pollution** | **Pollution** | **Brume + pollution** |
| **Donneur 1** | 17,99 | 24,32 | 3,44 | 1,15 | 15,00 | 8,74 | 3,02 | 2,38 |
| **Donneur 2** | 19,39 | 10,88 | 2,75 | 0,14 | 14,00 | 4,78 | 4,49 | 2,23 |
| **Donneur 3** | 9,97 | 13,24 | 5,33 | 2,47 | 20,98 | 12,79 | 1,45 | 1,73 |

Les deux polluants dosés dans cette étude ont été retrouvés de manière équivalente dans le lavage (non adsorbé). Ils ont été détectés de manière significativement plus faible dans le stratum corneum des explants pré-traités par la brume de Réotier. Cette protection contre la pénétration des polluants est également efficace dans l'épiderme après stripping (qui contient l'épiderme vivant et encore des couches de stratum corneum).

Ces résultats montrent que le traitement à l'eau de Réotier avant l'application de polluants permet d'en limiter la pénétration.

## Revendications

1. Utilisation cosmétique d'eau de Réotier pour limiter la pénétration cutanée d'un ou plusieurs agent(s) polluant(s) choisi(s) parmi un hydrocarbure, les composés organiques volatils, les métaux, et les particules fines ou ultrafines.

2. Utilisation selon la revendication 1, dans laquelle l'eau de Réotier est incluse dans une composition cosmétique comprenant de 1 à 100% en poids d'eau de Réotier.

3. Utilisation selon la revendication 2, dans laquelle la composition est appliquée sous la forme d'une brume.

4. Utilisation selon la revendication 2, dans laquelle la composition cosmétique comprend de 1 à 30% en poids d'eau de Réotier.

5. Utilisation selon l'une des revendications 1 à 4, dans laquelle ledit agent polluant est choisi parmi un hydrocarbure polycyclique aromatique (HPA), le plomb, le mercure, le cadmium, les particules de diamètre inférieur à 10 µm et les particules de diamètre inférieur à 2,5 µm.

## Patentansprüche

1. Kosmetische Verwendung von Réotier-Wasser zur Einschränkung der Hautdurchdringung eines Schadstoffs oder mehrerer Schadstoffe, ausgewählt aus einem Kohlenwasserstoff, flüchtigen organischen Verbindungen, Metallen und feinen oder ultrafeinen Partikeln.

2. Verwendung nach Anspruch 1, wobei das Réotier-Wasser in eine Kosmetikzusammensetzung eingebracht ist, die 1 bis 100 Gew.-% Réotier-Wasser umfasst.

3. Verwendung nach Anspruch 2, wobei die Zusammensetzung in Form eines Nebels aufgebracht wird.

4. Verwendung nach Anspruch 2, wobei die Kosmetikzusammensetzung 1 bis 30 Gew.-% Réotier-Wasser umfasst.

5. Verwendung nach einem der Ansprüche 1 bis 4, wobei der Schadstoff aus einem polyzyklischen aromatischen Kohlenwasserstoff (PAK), Blei, Quecksilber, Cadmium, Partikeln mit einem Durchmesser von weniger als 10 µm und Partikeln mit einem Durchmesser von weniger als 2,5 µm ausgewählt ist.

## Claims

1. Cosmetic use of Réotier water for limiting skin penetration of one or more pollutant agent(s) chosen from a hydrocarbon, volatile organic compounds, metals and fine or ultrafine particles.

2. Use as claimed in claim 1, wherein the Réotier water is included into a cosmetic composition comprising from 1 to 100% by weight of Réotier water.

3. Use as claimed in claim 2, wherein the composition is applied in the form of a mist.

4. Use as claimed in claim 2, wherein the cosmetic composition comprises from 1 to 30% by weight of Réotier water.

5. Use as claimed in one of claims 1 to 4, wherein said pollutant agent is chosen from a polycyclic aromatic hydrocarbon (PAH), lead, mercury, cadmium, particles less than 10 µm in diameter and particles less than 2.5 µm in diameter.
